# EUROPEAN PATENT APPLICATION

(11) **EP 4 454 669 A1**
(43) Date of publication of application: **30.10.2024**
(21) Application number: 24174943.1
(22) Date of filing: 28.04.2016
(51) Int. Cl.: A61L 27/16, A61L 27/30, A61L 27/32, A61K 6/00, A61K 6/17, A61K 6/76, A61K 6/887, A61K 6/851

(54) **METHOD FOR CREATING A MINERAL TRIOXIDE AGGREGATE MATERIAL WITH IMPROVED BIOLOGICAL EFFECTS**

(30) Priority: 29.04.2015 US 201562154282 P
(62) Divisional of application: 16720691.1
(71) Applicant: Dentsply Sirona Inc., York, PA 17401 (US)
(72) Inventor: WILKINSON, Kevin, Bixy (US); NDUNGU, Geoffrey, Tulsa (US)
(74) Representative: Wächtershäuser & Hartz Patentanwaltspartnerschaft mbB

(57) **Abstract**

A dental device is improved in its ability to produce hydroxyl apatite by having a layer of mineral trioxide aggregate (MTA) deposited thereon. A tile of MTA is prepared, heat treated and sintered to produce a micronized tile of MTA that can then be deposited by physical vapor depositions, hot isostatic pressing, molding or other conventional technique.

## Description

### Related U.S. Application Data

This application is a non-provisional claiming priority to pending provisional US Pat. App. Ser. No. 62/154,282 filed on April 29, 2015.

### Field of Invention

The present invention relates to the process of creating an improved biological Mineral Trioxide Aggregate (MTA) in a dense tile, or Target. The Target enables a process of coating the improved MTA on implantable medical devices or can be micronized into a MTA material for traditional use.

### Background of the Invention

Mineral trioxide aggregate (MTA) (sold under the trade names gray or white ProRoot MTA (Dentsply Sirona Inc., York, Pa., US) is a substance currently used in general dentistry and in endodontics to replace natural tooth material in apexification, pulp capping, pulpotomy, regenerative endodontics, root canal filling, root-end filling, root perforation repair, tooth restorations and the like. An example of MTA used in endodontic applications is disclosed in US Pat. No. 8,979,991 which is hereby incorporated by reference for such disclosure.

It would be desirable to provide an MTA material for dental applications including for endodontic, restorative or other uses, that has improved biological effect. More particularly, it would be beneficial to the dental arts to provide such a material that is demonstrated to improve the production of hydroxyl apatite (HA) in the presence of body fluids (or simulated body fluids such as phosphate buffered saline) to produce a material having cementum qualities.

For example, in endodontic applications, the material or composition should provide a stable barrier to bacteria and fluid leakage in the root canal system of a tooth. In addition, the composition should help promote the growth of the new bone and tissue surrounding the root tip area.

### Summary of the Invention

A dense MTA tile is produced through a series of process steps which creates a Target suitable for physical vapor deposition (PVD). The resulting MTA material has improved biological effects and produces HA at an accelerated rate when in the presence of simulated body fluid. The resulting Target can then be micronized or used to deposit a thin layer as desired, such as upon an obturation point. Conventional obturation points such as those made of gutta percha materials are useful in the application of the present invention.

According to one embodiment of the invention, a deposited thin layer of a cured or uncured MTA (Mineral Trioxide Aggregate) is deposited on the surface of otherwise conventional gutta-percha points. The material produces hydroxyl apatite (HA) mineral in the presence of Phosphate Buffered Saline which is beneficial in the formation of cementum like material for natural sealing of dental root canals.

In another embodiment cured MTA is deposited onto Gutta-percha points using a PVD method.

Another embodiment comprises the use of uncured MTA molded onto the surface of a gutta-percha point using a compression molding technique.

Still another inventive embodiment comprises the use of cured MTA compressed onto the surface of the gutta-percha using a compression molding technique.

A further embodiment of the invention includes the use of cured MTA molded onto the surface of the gutta-percha using an injection molding technique.

A still further embodiment comprises the use of uncured MTA molded onto the surface of the gutta-percha using an injection molding technique.

Another preferred embodiment is placing cured MTA onto implantable and prosthetic devices using PVD method

### Detailed Description of the Invention

A dense MTA with improved biological effects is produced through special processing techniques. The resulting dense MTA material has improved biological effects and in the presence of simulated body fluid and produces HA at an increased and accelerated rate.

Dense MTA targets can be created for use in physical vapor deposition (PVD) to provide a thin layer of MTA on the surface of implantable devices such as dental implants, endodontic obturation materials and the like. PVD techniques include:
**Cathodic Arc Deposition:** In which a high-power electric arc discharged at the target (source) material blasts away some into highly ionized vapor to be deposited onto the work piece.
**Electron beam physical vapor deposition:** In which the material to be deposited is heated to a high vapor pressure by electron bombardment in "high" vacuum and is transported by diffusion to be deposited by condensation on the (cooler) work piece.
**Evaporative deposition:** In which the material to be deposited is heated to a high vapor pressure by electrically resistive heating in "low" vacuum.
**Pulsed laser deposition:** In which a high-power laser ablates material from the target into a vapor.
**Sputter deposition:** In which a glow plasma discharge (usually localized around the "target" by a magnet) bombards the material sputtering some away as a vapor for subsequent deposition.

A thin layer of cured MTA (Mineral Trioxide Aggregate) is deposited on the surface of an implantable device.

The improved MTA is particularly suitable for obturating and sealing dental root canals. The improved MTA provides a stable barrier to bacterial and fluid leakage in the root canal. The accelerated generation of HA will also help promote the growth of new bone and tissue surrounding the root tip area. The improved MTA should also provide a stable barrier to bacteria and fluid leakage in the root canal system of the tooth.

The dense MTA material, Targets, may also be ground into small particles for use in Dental products or as a MTA powder with improved biological effects.

### Target Creation Process

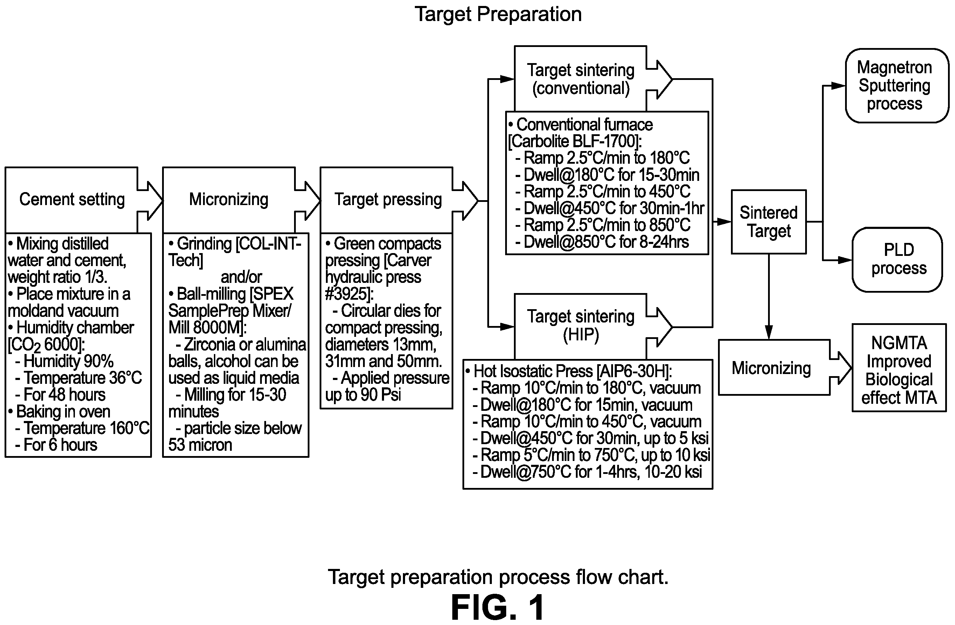

A method for forming the dense MTA material (Target) may include one or more of the steps/processes described below and further shown in Figure 1

### Mixing Process

Mixing (white) Portland Cement (WPC or PC) to Deionized water having a ratio in the range of about 10:1 to about 1:10, preferably about 5:1 to about 1:5, and more preferably about 5:1 to about 1:1 (e.g., about 3:1 such as about 3 parts PC (WPC) to about 1 part deionized water). The mixture is thoroughly blended and vacuum is drawn out to create a harmonized cake mix and then placed in molds.

### Curing process

Once the mixed cement is put in molds, the molds are placed in a humidity chamber to cure. The humidity chamber is set at 36°C with 90% Relative Humidity (RH) from about 5 hours to about 10 days, and preferably from about 12 hours to about 5 days (e.g., about 2 days).

### Post Cure Baking Process

Once the cement is cured, the set cement is placed in a Post cure bake oven set at a temperature from about 50°C to about 500°C, and preferably from about 100°C to about 250°C (e.g., about 160°C) for a time ranging from about 15 mins to about 2 days, and preferably from about 1 hour to about 12 hours (e.g., about 6 hours).

### Ball Milling and Micronizing Process

The target creation process may include a micronizing step. Once the cement is post baked, it is then pulverized, micronized and sieved to a particle size ranging from about 1 micron to about 200 microns, and preferably from about 10 microns to about 100 microns (e.g., about 53 microns (µ53)).

### Sintering Process

The target creation process may include a Sintering Step. The Powder Micronized MTA is pressed into a target then placed into a sintering chamber for a first heat treatment at a temperature ranging from about 25°C to about 400°C, preferably from about 50°C to about 250 degrees Celsius (e.g., about 125°C) for a time period of about 5 mins to about 5 hours, preferably from about 15 mins to about 2 hours (e.g., about 30 to about 40 mins) at little or substantially no pressure. Optionally, the sintering process may include a second heat treatment, though not required. When included, the temperature may be increased to a third heat treatment temperature that is higher than the first heat treatment temperature, the second heat treatment temperature ranging from about 275°C to about 650°C, and preferably from about 350°C to about 525°C (e.g., about 450°C) for a time period ranging from about 5 minutes to about 12 hours, and preferably from about 15 mins to about 5 hours (e.g., about 1 to 2 hours) at a pressure ranging from about 5000 pounds/in² (psi) to about 50,000 psi, and preferably from about 10,000 psi to about 25,000 psi (e.g., about 15,000 psi). Optionally, the sintering process may include a third heat treatment, though not required. When included, the heat treatment temperature of the sintering process may be increased to a third heat treatment temperature that is higher than the second heat treatment temperature, the third heat treatment temperature ranging from about 550°C to about 1100°C, and preferably from about 650°C to about 975°C (e.g., about 750°C to 850°C) for a period of time ranging from about 30 mins to about 2 days, and preferably from about 2 hours to about 1 day (e.g., about 4 hours to about 12 hours) at a pressure ranging from about 1,000 psi to about 50,000 psi, and preferably from about 7,500 psi to about 35,000 psi (e.g., about 15,000 psi to 20,000 psi).

### HIP Process

The target creation process may include a hot isostatic pressing (HIP) step/process. The set sintered targets may be placed into a HIP chamber at a temperature ranging from about 250°C to about 1500°C, and preferably from about 500°C to about 1000°C (e.g., about 750°C to about 850°C) for a period of time ranging from about 30 mins to about 2 days, and preferably from about 2 hours to about 1 day (e.g., about 4 hours to about 12 hours) at a pressure ranging from about 1,000 psi to about 50,000 psi, and preferably from about 7,500 psi to about 35,000 psi (e.g., about 15,000 psi to 20,000 psi).

### Post HIP, NGMTA Improved Biological Effect MTA

The HIPped targets may be micronized to a particle size ranging from about 1 micron to about 200 microns, and preferably from about 10 microns to about 100 microns (e.g., about 53 microns (µ53)) wherein the resultant powder MTA has improved biological effects and produces HA at an accelerated rate.

### Ranges of Parameters

The experimented parameters for one or more of the above steps and/or processes may range from:
The temperature(s) may range from about 25°C to about 1500°C, preferably from about 500°C to about 1200°C.

The pressure(s) may range from about 0 psi to about 50,000 psi, preferably from about 15,000 psi to about 20,000 psi.

The time periods may range from about 5 mins to about 10 days, preferably from about 2 hours to about 5 hours.

The preferred method of coating medical devices with MTA is physical vapor deposition (PVD), using magnetron sputtering. Alternative methods of Physical Vapor Deposition may also include Pulsed Laser Deposition (PLD), Magnetron Sputtering, and the like as above discussed

Other useful techniques for coating medical devices with MTA include:
Compression molding which entails sprinkling MTA on the substrate during compression molding process; and,
Micronized powder and spraying, this entails blasting the substrate with high velocity spray of MTA.

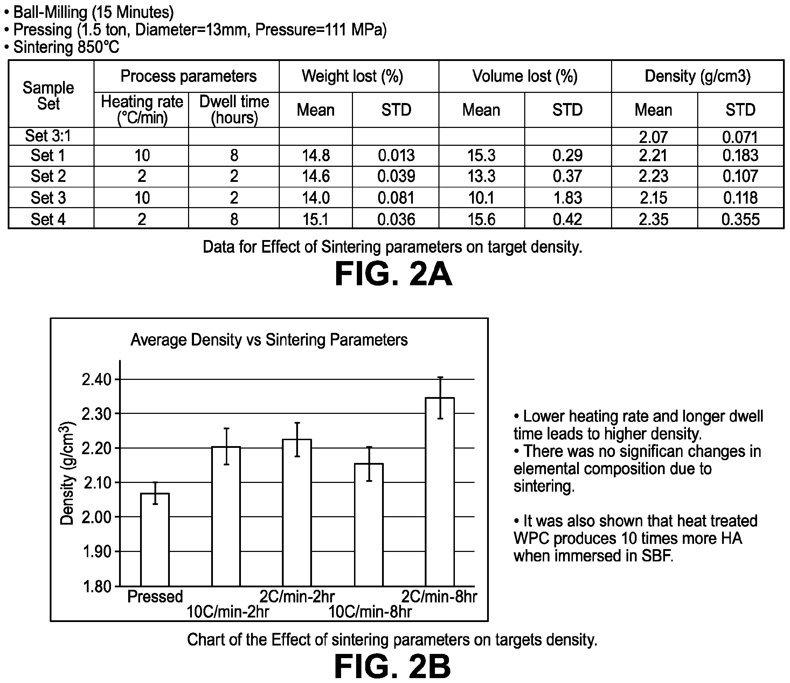

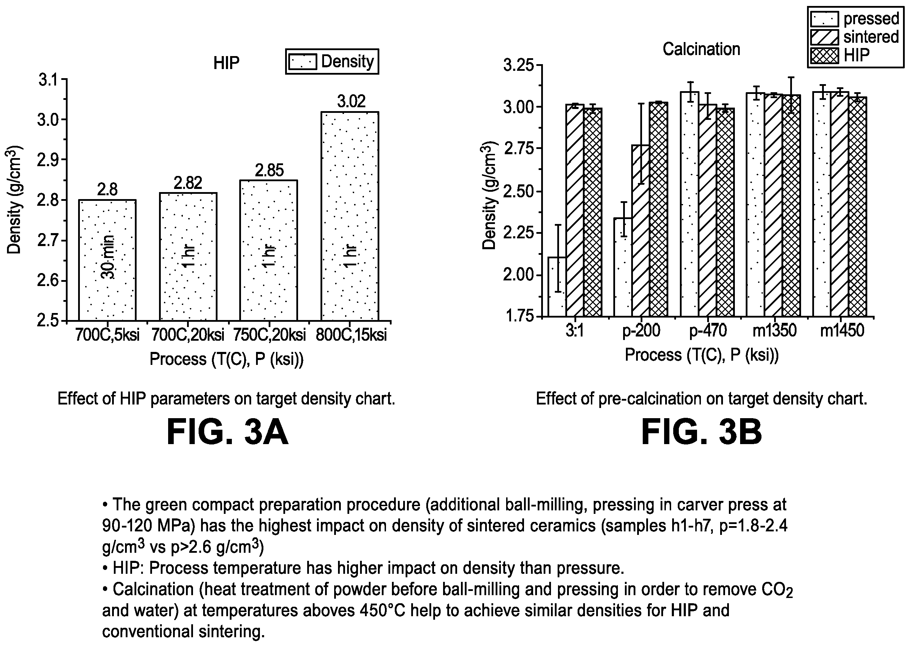

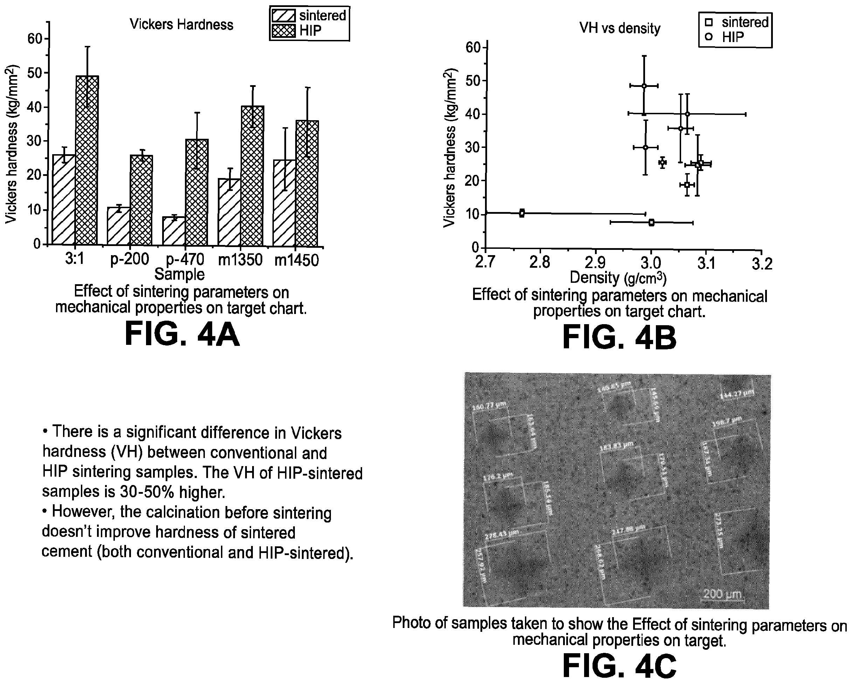

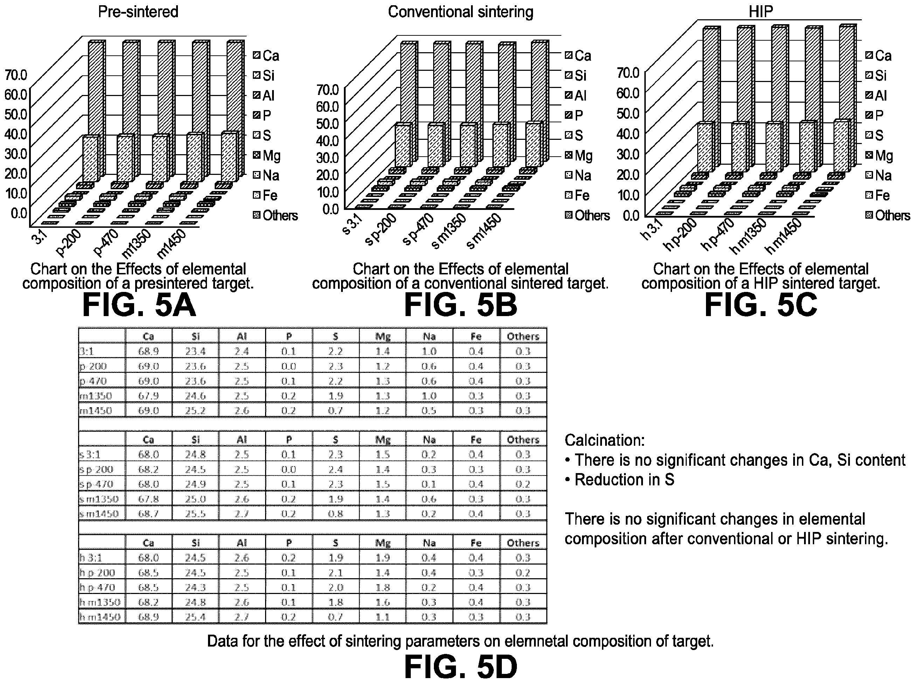

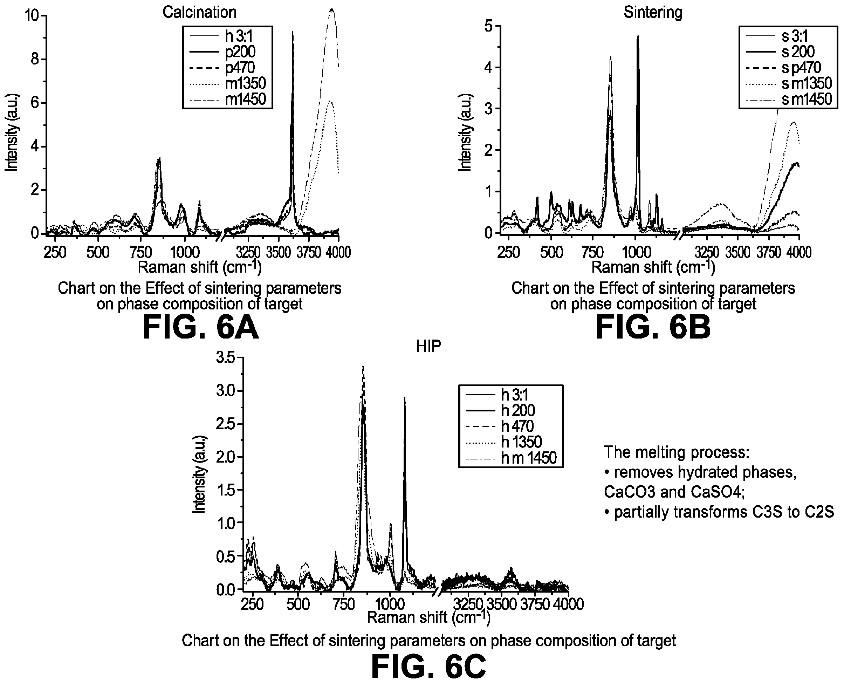

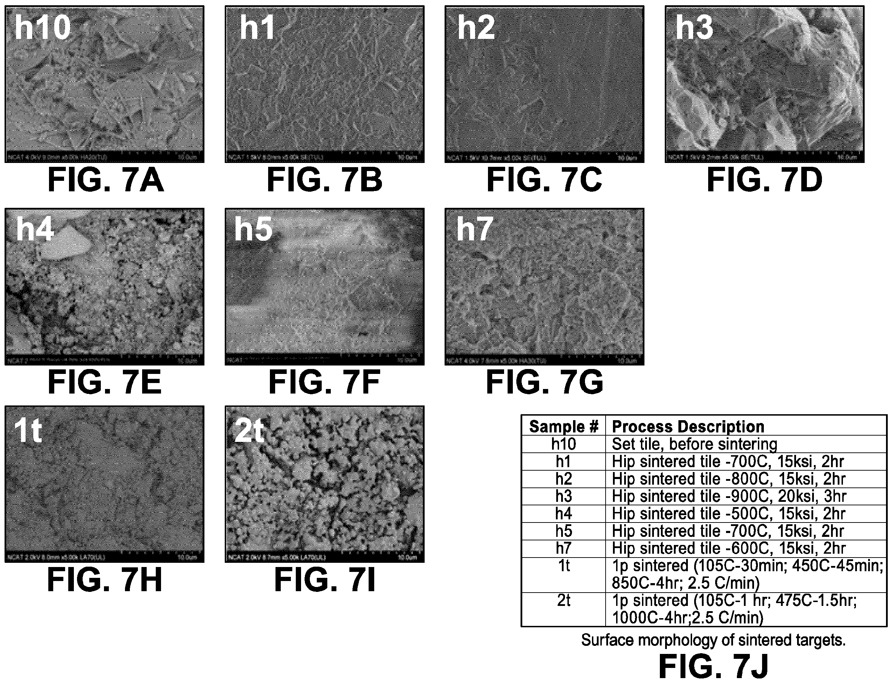

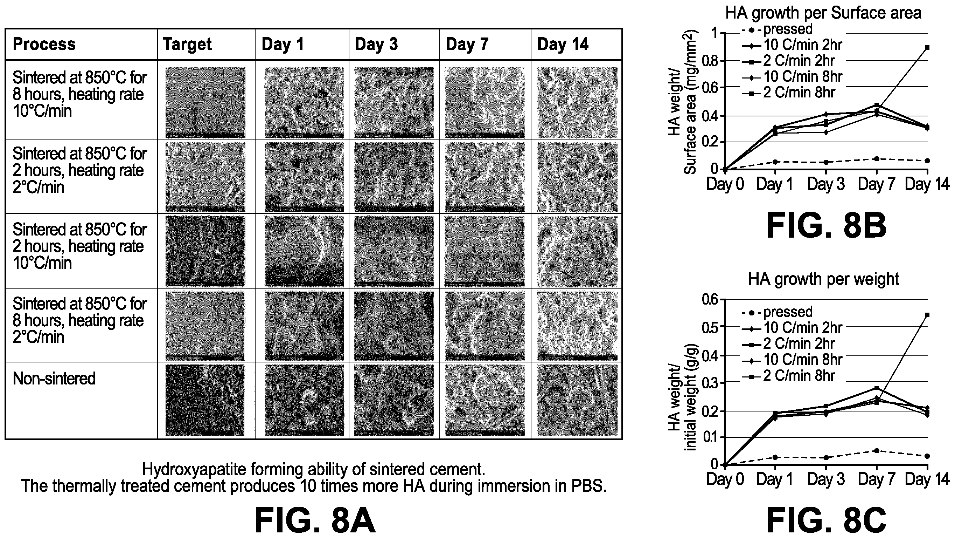

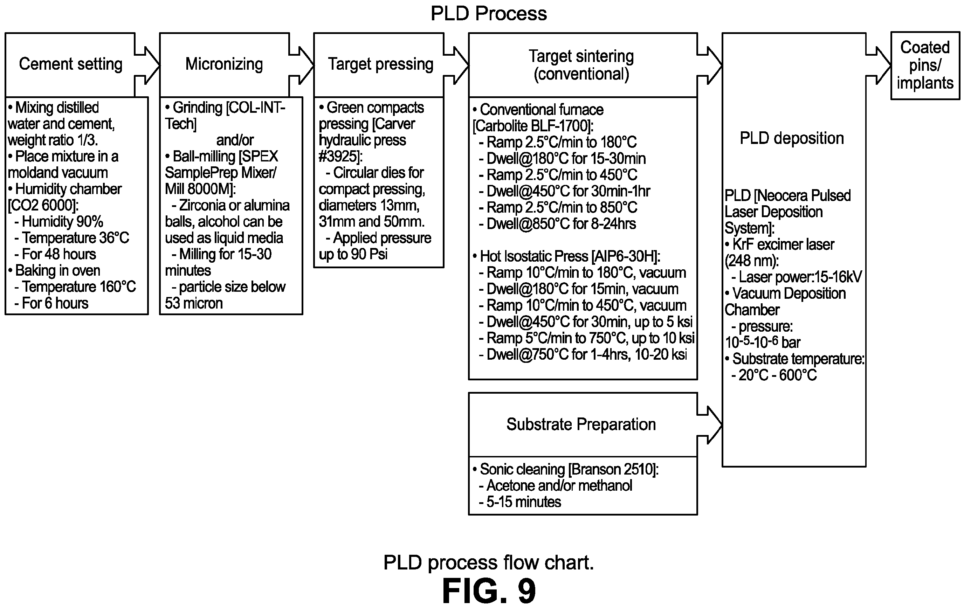

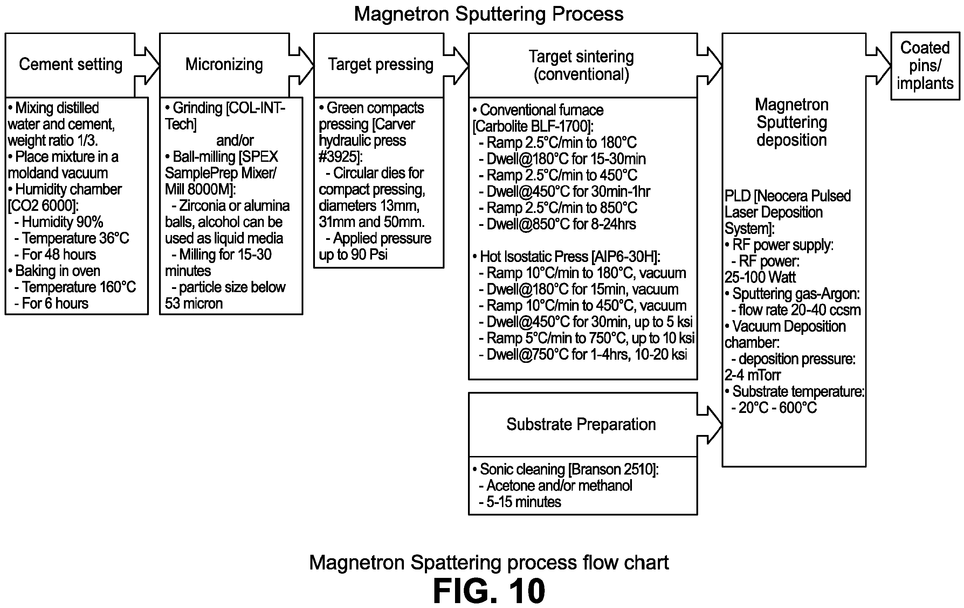

In one example of the invention, optimum parameters for a dense target producing increased HA generation (Figure 8) include a heat treatment profile having a first heat treatment of about 120°C at about 0 psi for about 1 hour, a second heat treatment of about 450°C at about 15,000 psi for about 2 hours, and a third heat treatment of about 850°C at about 20,000 psi for about 4 hours (Figures 2-7).

### Pulsed Laser Deposition (PLD)

This is a thin film deposition technique where a high-power pulsed laser beam is focused inside a vacuum chamber to strike a target of the material that is to be deposited. This material is vaporized from the target (in a plasma plume) which deposits it as a thin film on the substrate (in this case the GP points); this process occurs in ultra-high vacuum.

While the basic-setup is simple relative to many other deposition techniques, the physical phenomena of laser-target interaction and film growth are quite complex. When the laser pulse is absorbed by the target, energy is first converted to electronic excitation and then into thermal, chemical and mechanical energy resulting in evaporation, ablation, plasma formation and even exfoliation. The ejected species expand into the surrounding vacuum in the form of a plume containing many energetic species including atoms, molecules, electrons, ions, clusters, particulates and molten globules, before depositing on the substrate.

### Magnetron Sputtering

High Power Impulse Magnetron Sputtering (HIPIMS or HiPIMS, also known as high-power pulsed magnetron sputtering, HPPMS) is a method for physical vapor deposition of thin films which is based on magnetron sputter deposition. HIPIMS utilizes extremely high power densities of the order of kW·cm⁻² in short pulses (impulses) of tens of microseconds at low duty cycle (on/off time ratio) of < 10%.

Distinguishing features of HIPIMS are a high degree of ionisation of the sputtered material and a high rate of molecular gas dissociation which result in high density of deposited films. The ionization and dissociation degree increase according to the peak cathode power. The limit is determined by the transition of the discharge from glow to arc phase. The peak power and the duty cycle are selected so as to maintain an average cathode power similar to conventional sputtering (1-10 W·cm⁻²).

By using magnets behind the cathode to trap the free electrons in a magnetic field directly above the target surface, these electrons are not free to bombard the substrate to the same extent as with diode sputtering. At the same time the extensive, circuitous path carved by these same electrons when trapped in the magnetic field, enhances their probability of ionizing a neutral gas molecule by several orders of magnitude. This increase in available ions significantly increases the rate at which target material is eroded and subsequently deposited onto the substrate.

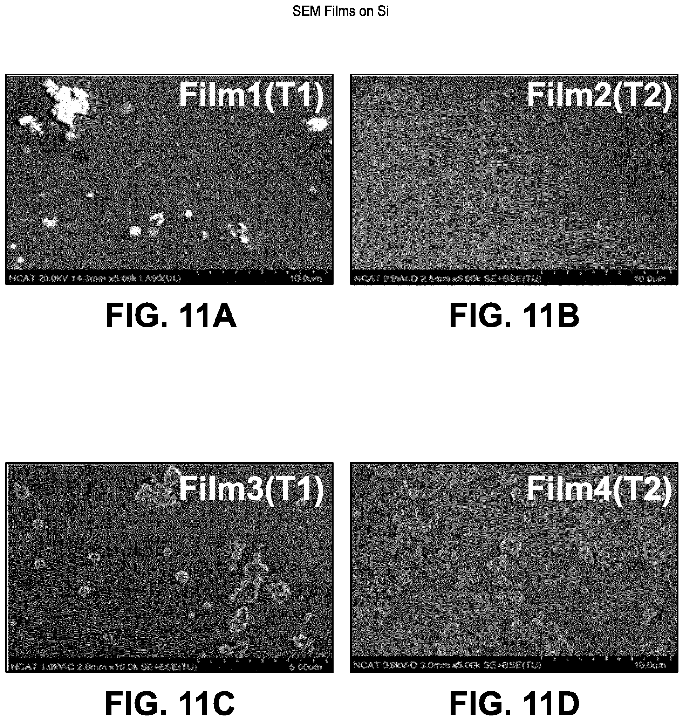

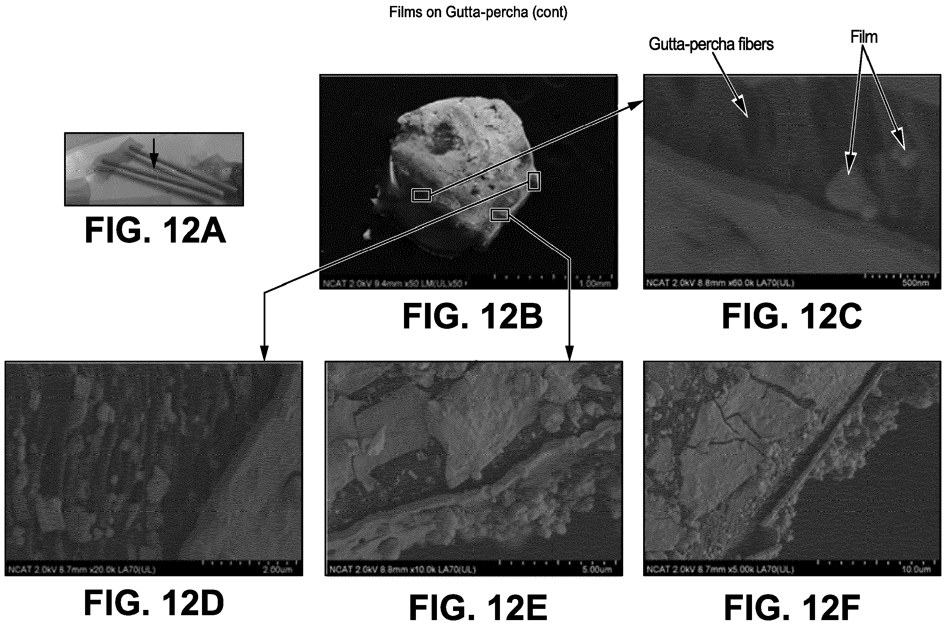

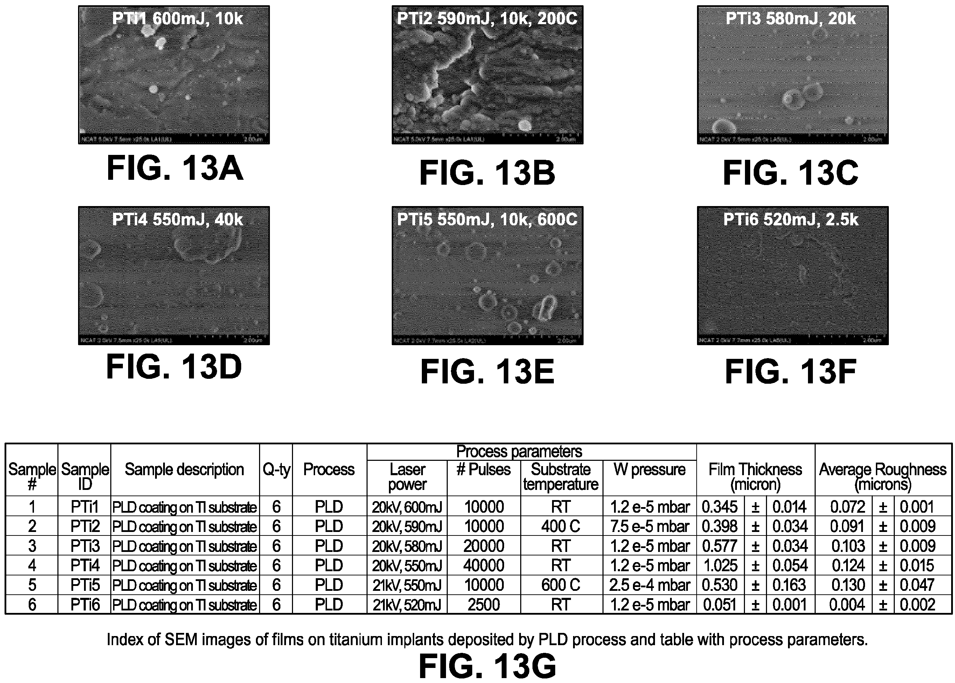

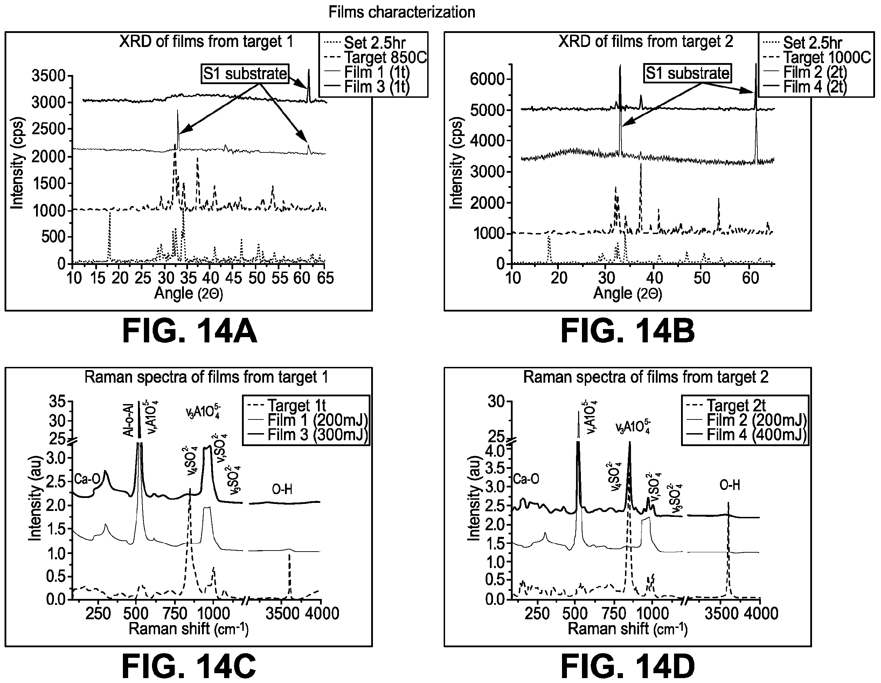

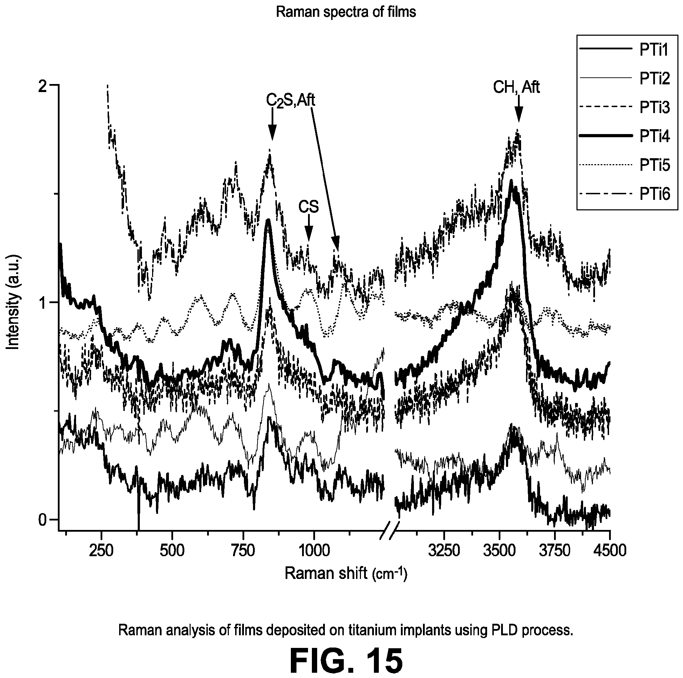

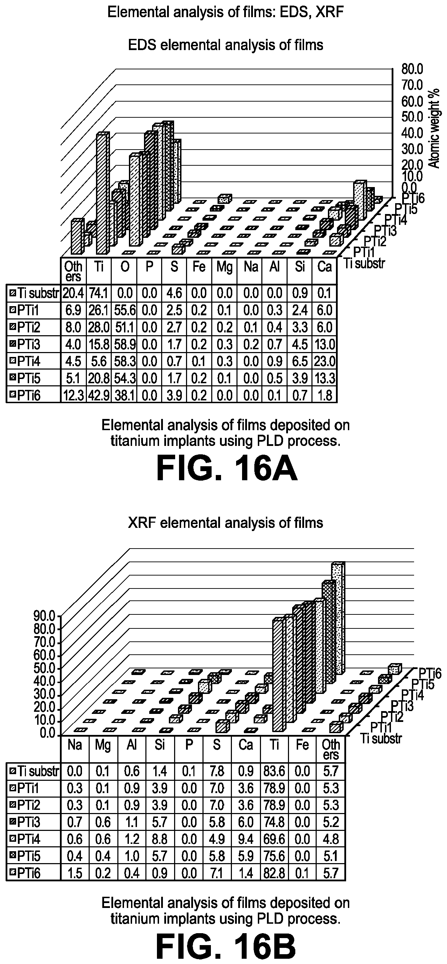

MTA films were deposited on Silicone, Gutta Percha and Titanium samples using PVD. The thickness and concentration can be altered based on duration and power levels (Figures11-13). Characterization of raw material sintering of targets for Pulsed Laser Deposition (PLD) and deposition of material in the form of thin films on testing substrates, gutta-percha and titanium implants (Figures 13-16). To ensure that physical vapor deposition (PVD) does not change chemical composition of raw materials, the full materials characterization of WPC (White Portland Cement) included elemental analysis by electron dispersive spectroscopy (EDS, figure 16), phase analysis by X-ray diffractometry (XRD, figure 14), composition analysis using (micro-Raman, figures 14, 15), density measurements and morphological studies by scanning electron microscopy (SEM, figures 11-13).

### Deposition of films on Alternate Materials

PVD can also be used to deposit a thin layer of MTA on a variety of surfaces. Feasibility has been established for PVD of MTA on polyisoprene and ceramic surfaces. Other potential applications include deposition of a MTA film on variety of metal or ceramic surfaces such as NiTi, Titanium, stainless steel, and porcelain.

From HIP-Sintered WPC targets supplied Pulsed Laser Deposition PLD was performed and uniformity of thickness and composition of deposited material was studied on Implantable and prosthetic devices.

### HA (hydroxyapatite) formation Study

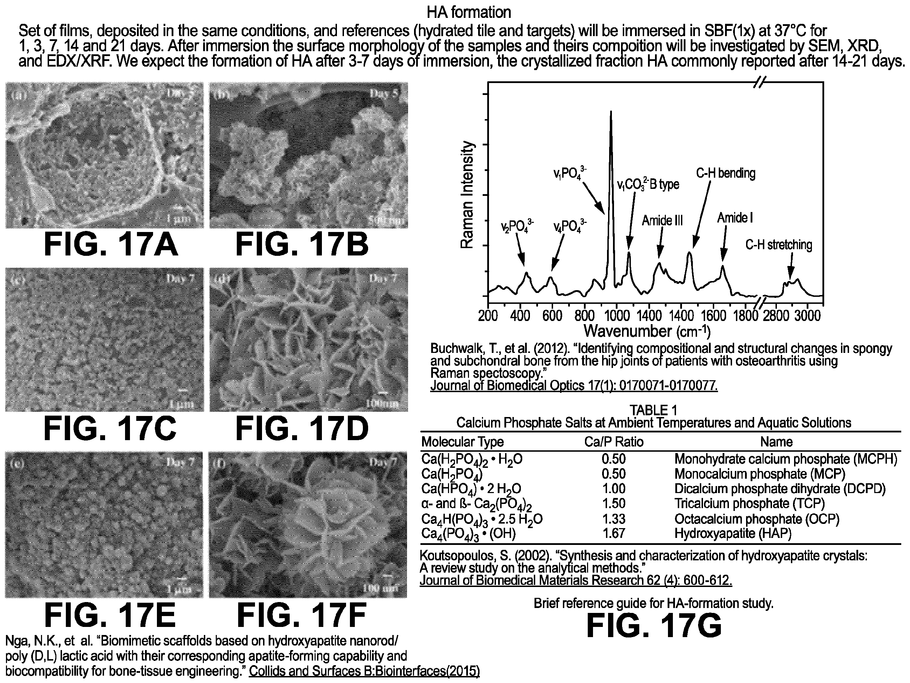

In order to confirm bio-activity of MTA coating the HA-forming ability was tested on films deposited on silicon, gutta-percha and titanium. The deposited films were soaked in simulated body fluids (SBF) at 37°C for 1, 3, 7, 14 and 21 days. The HA formation was observed after 1 day, whereas typically HA formation is not first observed until 7 days. (Figure 17).

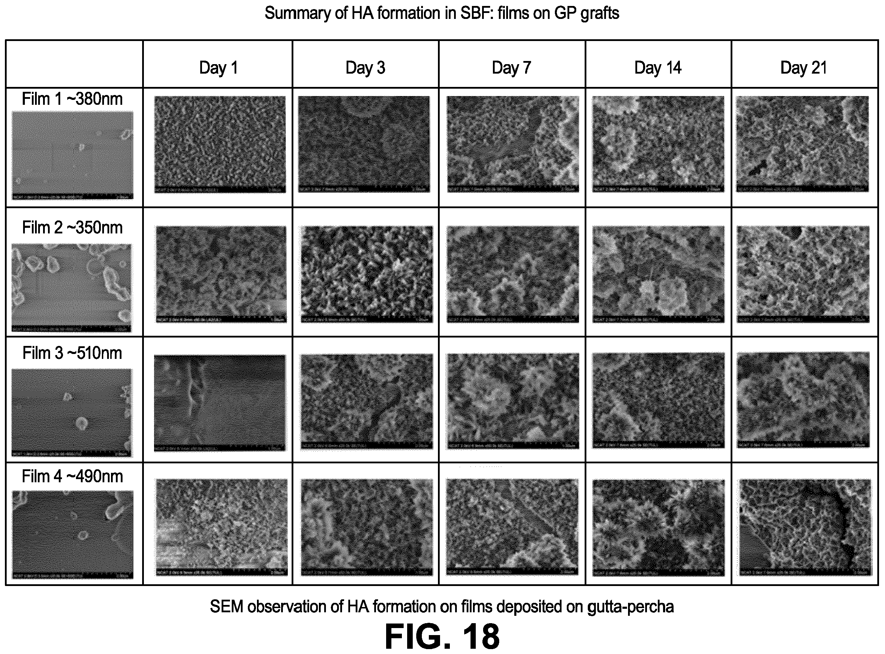

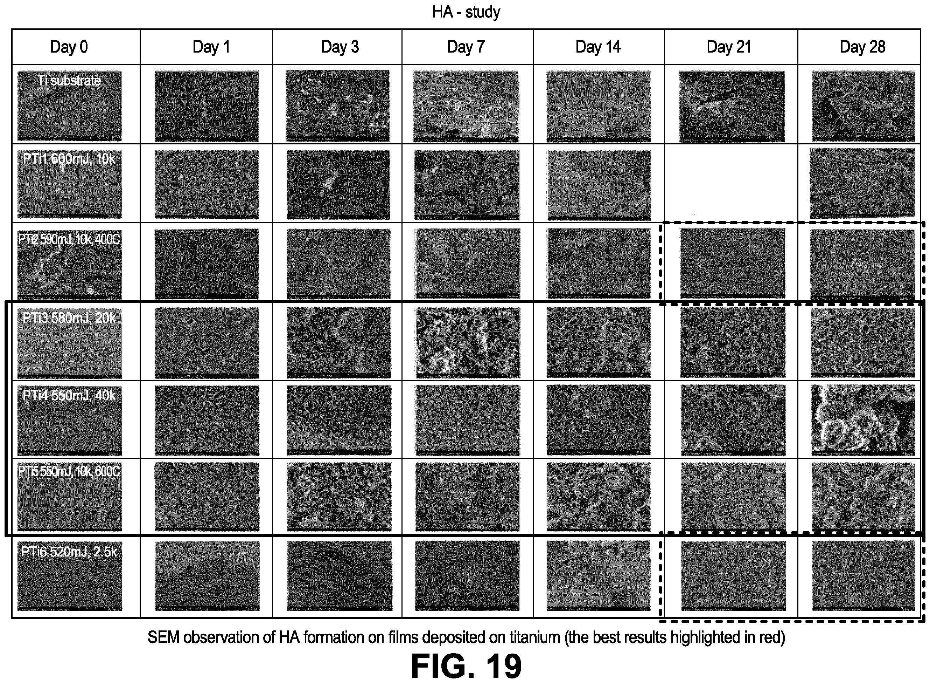

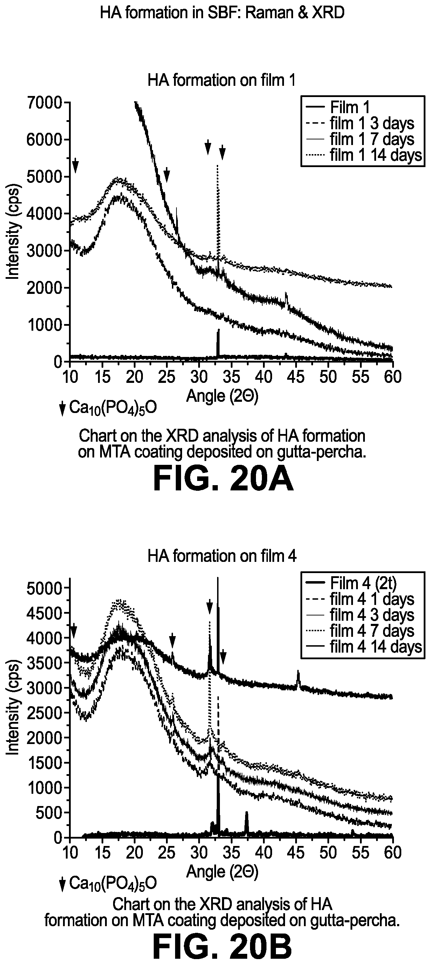

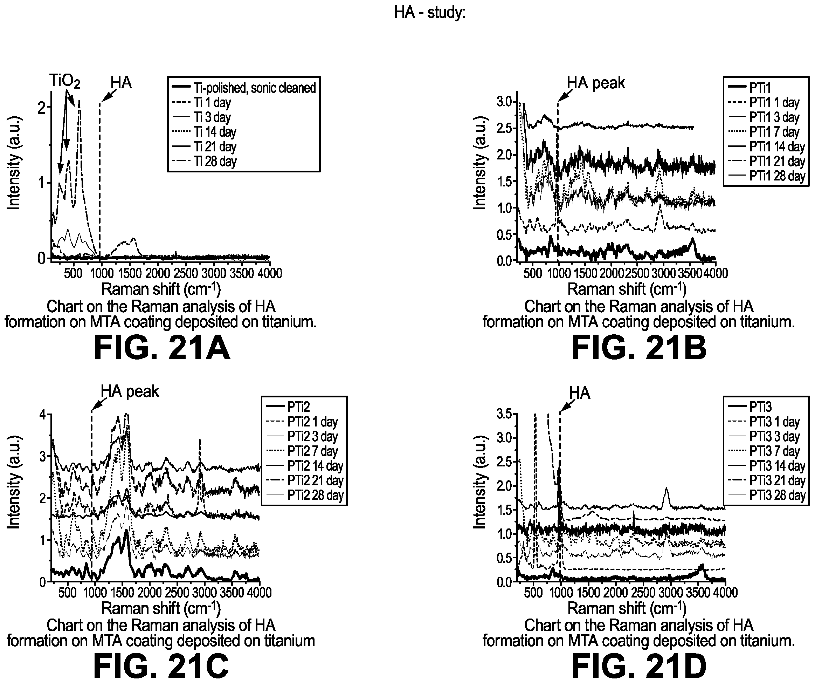

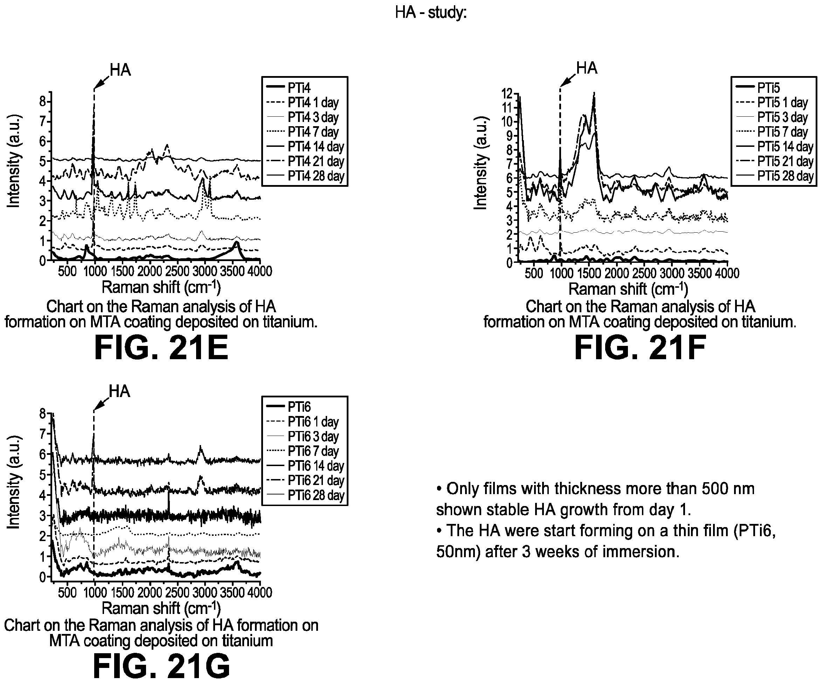

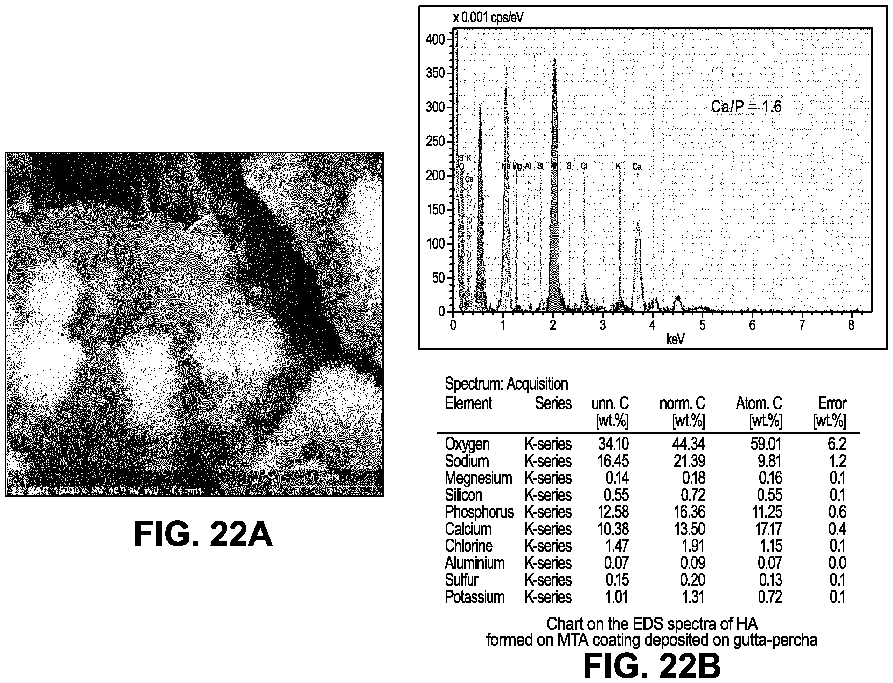

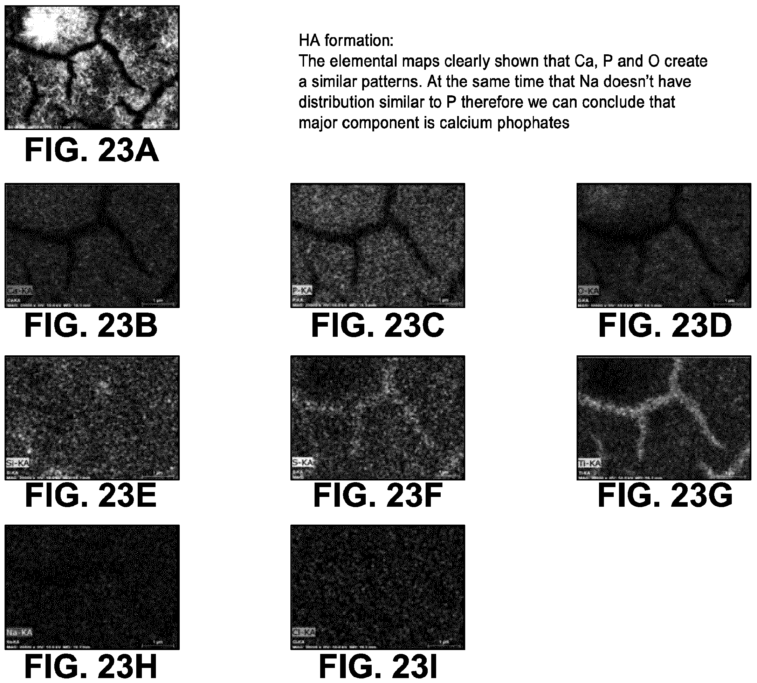

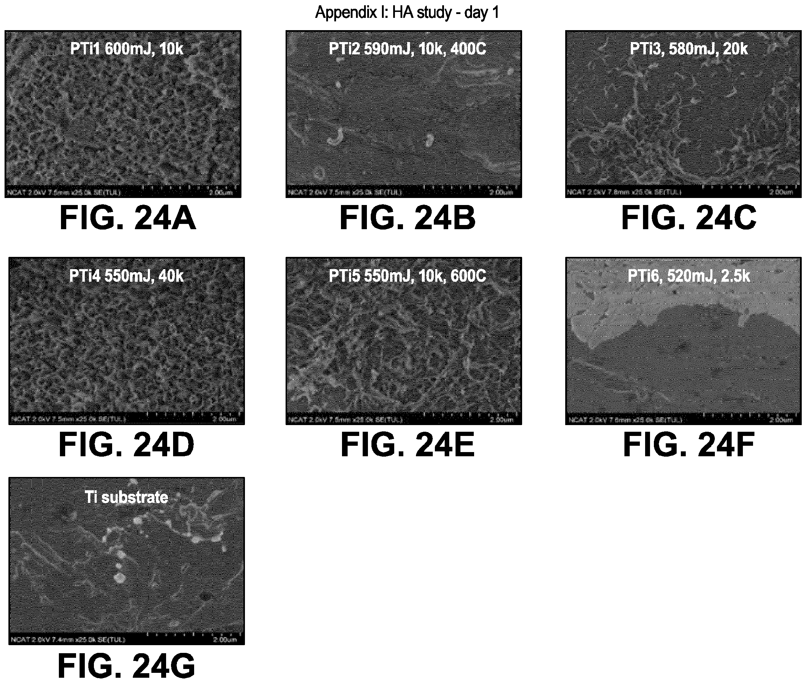

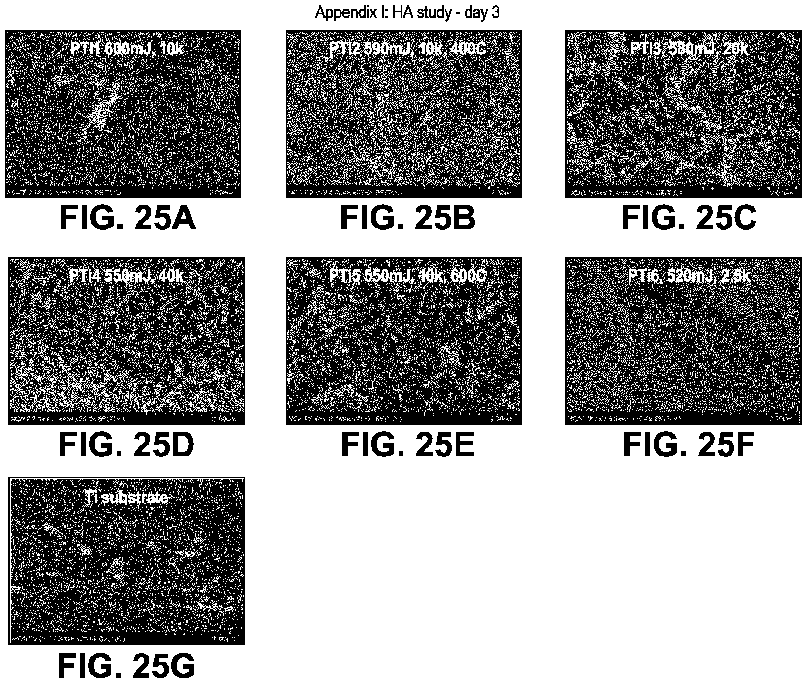

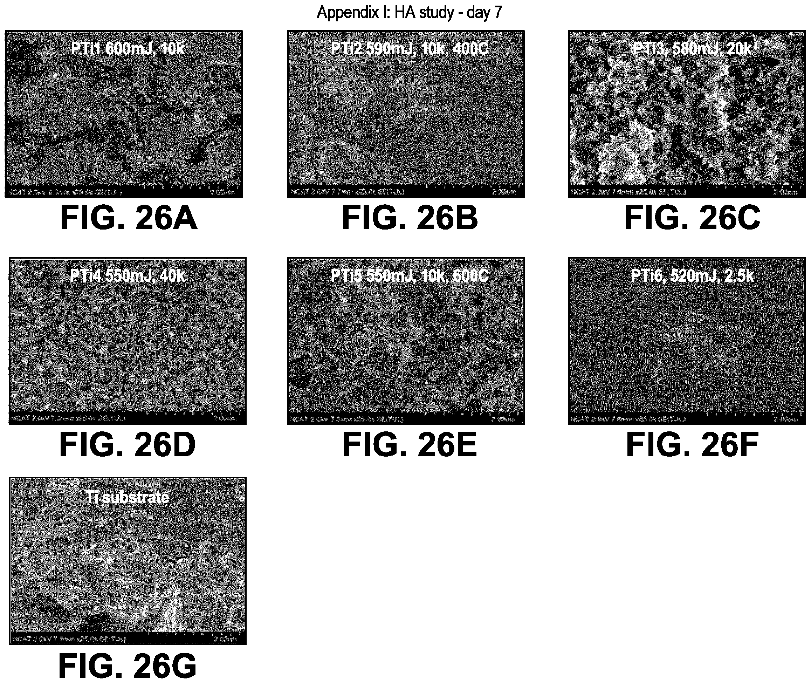

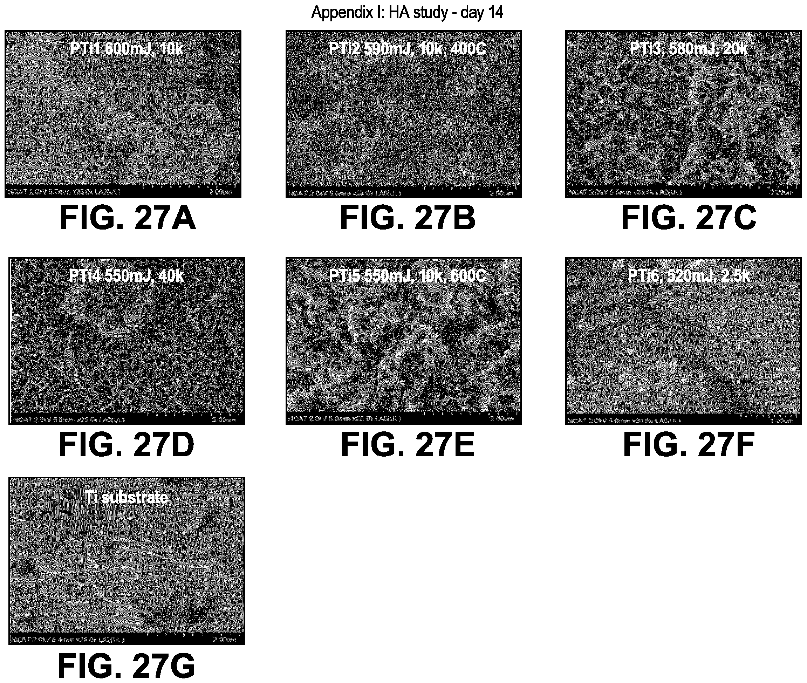

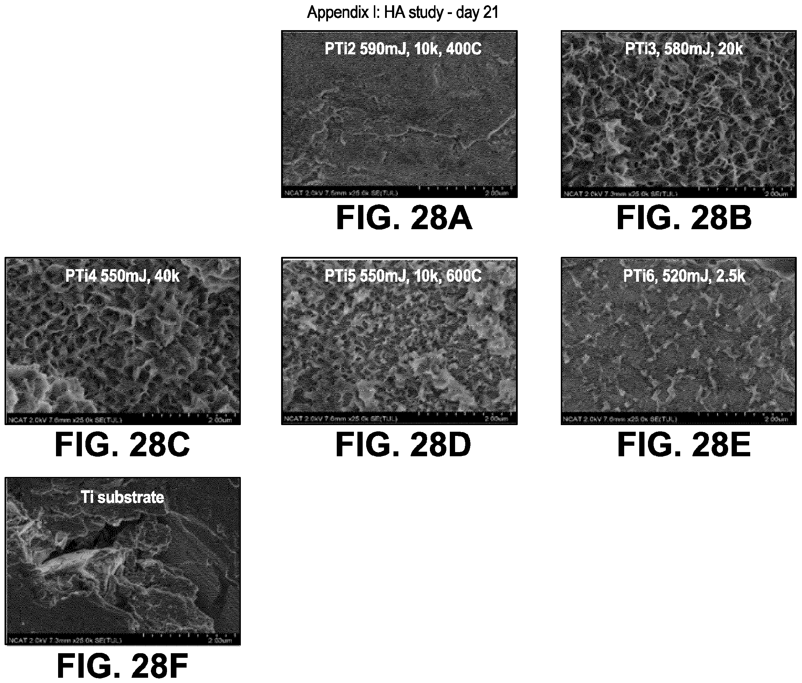

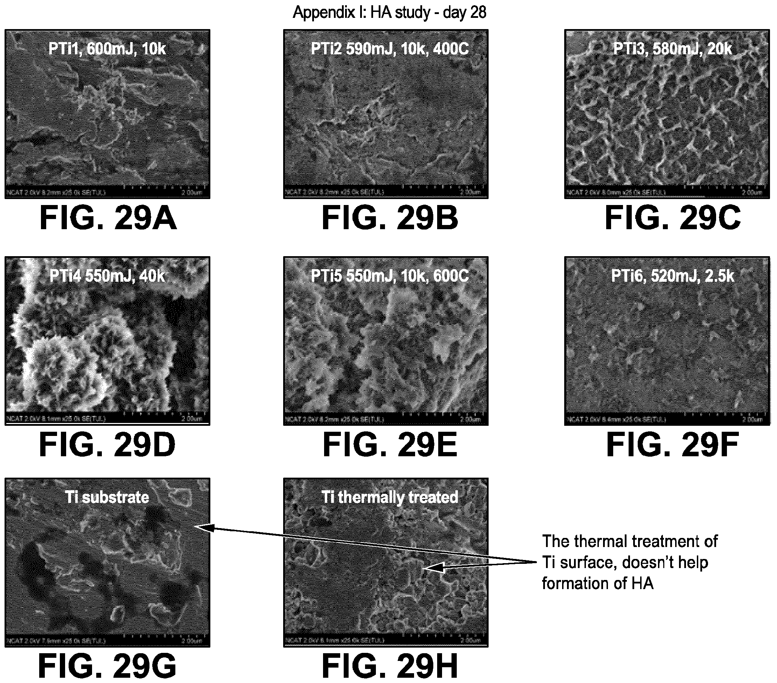

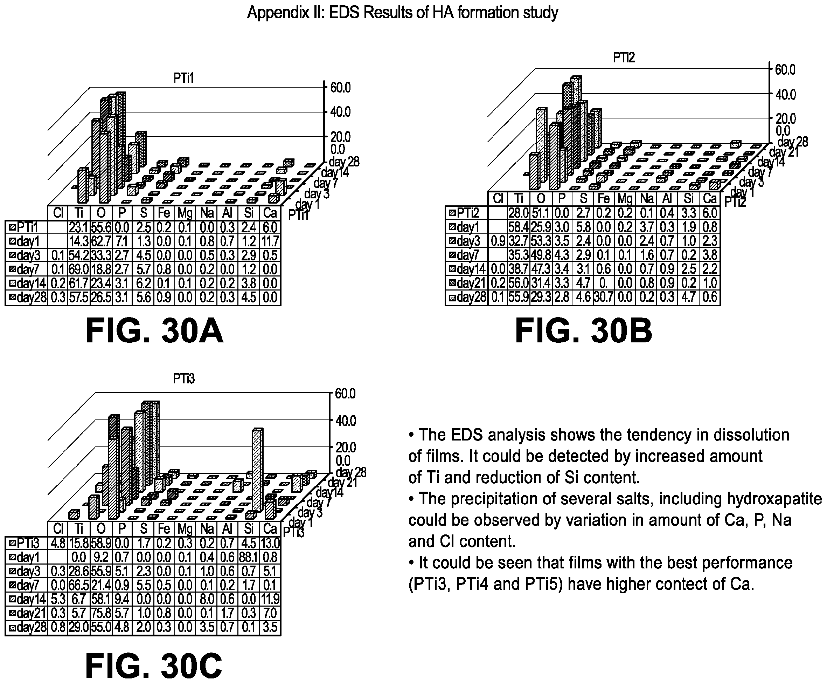

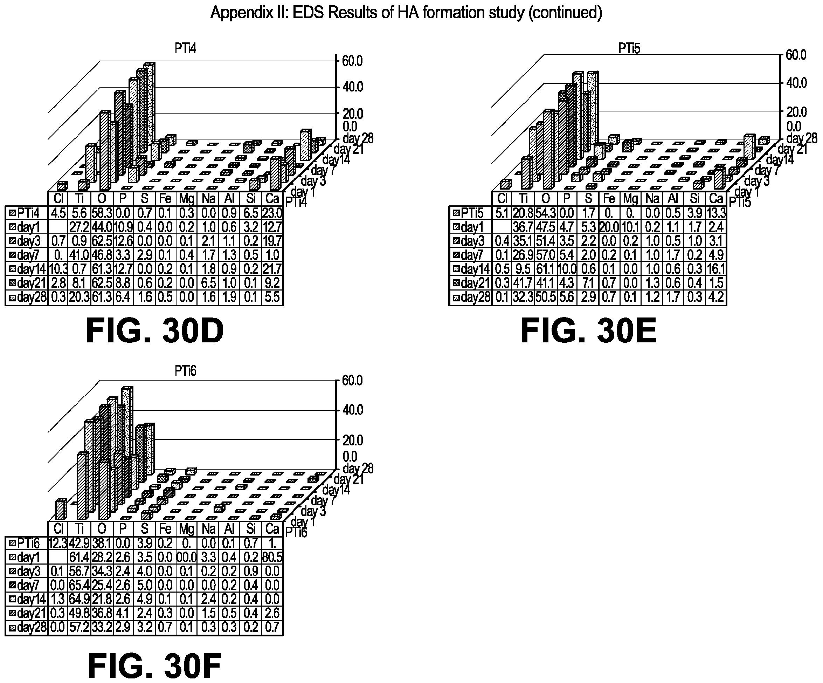

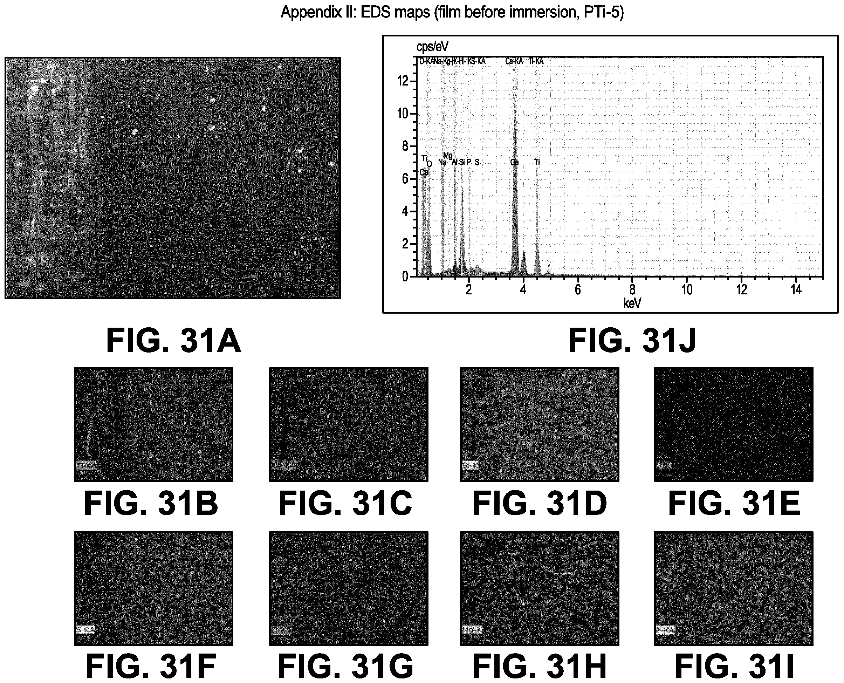

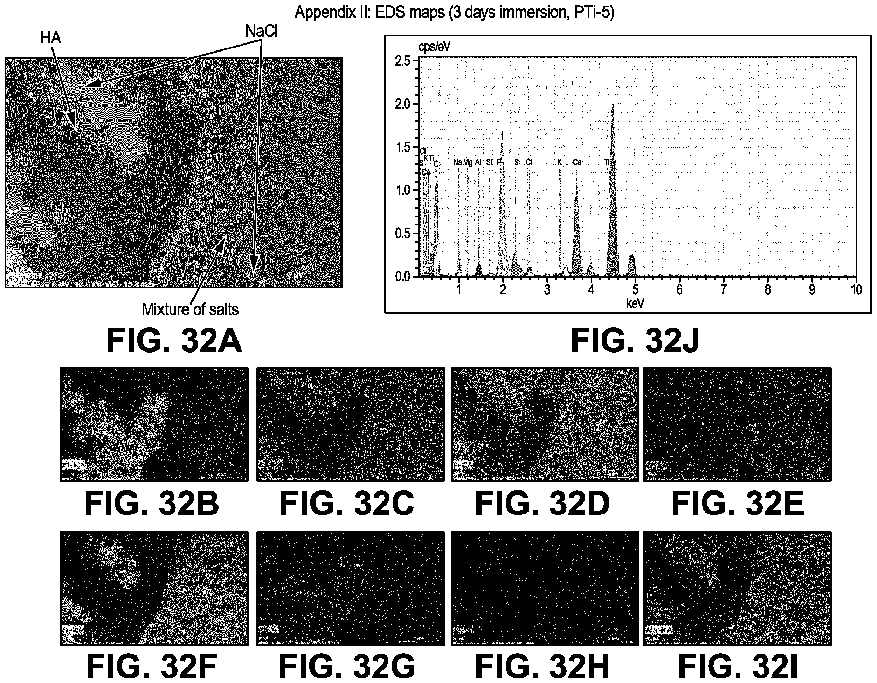

The formation of hydroxyl apatite (HA) and other phosphates were confirmed by SEM imaging (figures 18, 19) and analyzed by XRD and micro-Raman (figures 20, 21). The Ca/P ratio of produced HA was studied by EDS and newly acquired x-ray fluorescence (XRF) (figures 22, 23).

Thus, it should be evident that the invention as disclosed herein carries out one or more of the objects of the present invention set forth above and otherwise constitutes an advantageous contribution to the art. As will be apparent to persons skilled in the art, modifications can be made to the preferred embodiments disclosed herein without departing from the spirit of the invention, the scope of the invention herein being limited solely by the scope of the attached claims.

## Claims

1. An implantable dental device comprising a layer of dense mineral trioxide aggregate that produces hydroxyl apatite in the presence of phosphate buffered saline.

2. An implantable dental device as in claim 1 wherein said device is an obturation point.

3. An implantable device as in claim 2 wherein said obturation point comprises gutta-percha.

4. A method for producing hydroxyl apatite on a dental device comprising the step of preparing a tile of dense mineral trioxide aggregate.

5. A method as in claim 4 further comprising forming a mixture by mixing Portland cement and deionized water in an amount of from about 10:1 to about 1:10.

6. A method as in claim 5 wherein said mixture is placed into a mold and cured in a humidity chamber.

7. A method as in claim 6 wherein said humidity chamber is set at 36 degrees Celsius with about a 90 percent relative humidity for from about 5 hours to about 10 days.

8. A method as in claim 7 wherein said cured mixture is subjected to a second heating by heating to from about 50 to about 500 degrees Celsius for from about 15 minutes to about 2 days.

9. A method as in claim 8 wherein said method includes micronizing said cured tile.

10. A method as in claim 9 wherein said micronizing includes ball-mill grinding said tile to a particle size of from about 1 to about 200 microns.

11. A method as in claim 10 wherein said micronizing includes ball-mill grinding said tile to a particle size of from about 10 to about 100 microns, preferably wherein said micronizing includes ball-mill grinding said tile to a particle size of about 53 microns.

12. A method as in claim 9 wherein said micronized tile is sintered at a temperature of from about 25 to about 400 degrees Celsius for a period of from about 5 minutes to about 5 hours.

13. A method as in claim 11 wherein said sintered, micronized tile is subjected to a second sintering temperature of from about 275 to about 650 degrees Celsius for from about 5 minutes to about 12 hours, preferably wherein said second sintering step is conducted at a pressure of from about 5000psi to about 50,000psi.

14. A method as in claim 13 wherein said sintered, micronized tile is subjected to a third sintering temperature of from about 550 to about 1100 degrees Celsius for from about 30 minutes to about 2 days, preferably wherein said third sintering step is conducted at a pressure of from about 1000psi to about 50,000psi.

15. A method as in claim 9 wherein said step of depositing includes physical vapor deposition of said micronized tile; or wherein said step of depositing includes hot isostatic pressing of said micronized tile; or wherein said step of depositing includes hot isostatic pressing of said micronized tile; or wherein said step of depositing includes molding of said micronized tile.
